# EUROPEAN PATENT APPLICATION

(11) **EP 4 803 008 A1**
(43) Date of publication of application: **09.09.2026**
(21) Application number: 25161647.0
(22) Date of filing: 04.03.2025
(51) Int. Cl.: A61B 6/04, A61B 5/00, A61B 6/46, A61B 6/00

(54) **RADIOGRAPHY APPARATUS**

(71) Applicant: Koninklijke Philips N.V., 5656 AE Eindhoven (NL)
(72) Inventor: KROENKE-HILLE, Sven, Eindhoven (NL); MENSER, Bernd, Eindhoven (NL); BYSTROV, Daniel, 5656AG Eindhoven (NL); VON BERG, Jens, Eindhoven (NL); MAY, Jan Marek, Eindhoven (NL); WIEBERNEIT, Nataly, Eindhoven (NL); HAWELLEK, Benjamin, Eindhoven (NL)
(74) Representative: Philips Intellectual Property & Standards

(57) **Abstract**

The present disclosure describes a radiography apparatus (100). The apparatus comprises a radiographic imager (101) configured to capture a radiographic image of a target, the radiographic imager comprising a radiation generator (102) and a first image sensor (104). The apparatus comprises a positional imager comprising a second image sensor configured to capture a positioning image of the target (such as a visible light image). The apparatus comprises a controller (122) configured to control a first data acquisition comprising controlling the second image sensor to capture a first positional image of the target at substantially the same time as controlling the radiographic imager to capture a first radiographic image of the target, and to store the captured first images in a memory. The apparatus comprises a display, wherein prior to a second data acquisition, the controller controls the display to display a user interface comprising displaying a live image (132) of the target currently captured by the second image sensor, the first positional image (134) and the first radiographic image (136).

## Description

### FIELD OF THE INVENTION

The subject-matter of the present disclosure relates to radiography, and in particular obtaining accurate images from a radiography apparatus and radiography apparatus therefor.

### BACKGROUND OF THE INVENTION

X-ray imaging requires proper patient positioning relative to the radiography system for acquiring images of diagnostic quality. For musculoskeletal exams, achieving an appropriate patient pose is so difficult that often several image acquisitions are required until the positioning is of sufficient quality. One key issue is that the radiographer only observes the quality of the positioning on the acquisition workstation upon observing the captured X-ray. This is often done in a control room separate to an acquisition room in which the X-ray is captured. The radiographer must therefore use this information to improve the patients pose whilst also travelling back and forth between control and acquisition rooms. This approach to X-ray reacquisition causes delay and also has high likelihood of not achieving the correct patient pose.

It is therefore an aim of the subject-matter of the present disclosure to improve on the prior art.

### SUMMARY OF THE INVENTION

According to a first aspect of the present invention, there is provided a radiography apparatus. The apparatus comprises a radiographic imager configured to capture a radiographic image of a target, the radiographic imager comprising a radiation generator and a first image sensor. The apparatus comprises a position imager comprising a second image sensor configured to capture a positioning image of the target (such as a visible light image). The apparatus comprises a controller configured to control a first data acquisition comprising controlling the second image sensor to capture a first positional image of the target at substantially the same time as controlling the radiographic imager to capture a first radiographic image of the target, and to store the captured first images in a memory. The apparatus comprises a display, wherein prior to a second data acquisition, the controller controls the display to display a user interface comprising displaying a live image of the target currently captured by the second image sensor, the first positional image and the first radiographic image.

In an example, displaying the first radiographic image may comprise overlaying the first radiographic image and a corresponding area of the first positional image.

In an example, the positional imager may comprise a depth sensor.

In an example, the user interface may comprise displaying a three dimensional rendering of the first visible spectrum image on top of the first radiographic image.

In an example, the user interface may comprise displaying the currently captured live image overlaid with first visible spectrum image.

In an example, the controller may be configured to determine a spatial correspondence of the first image sensor with respect to the live image being captured.

In an example, the first image sensor may comprise one or more markers, and determining the spatial correspondence of the first image sensor may comprise determining the location of the markers within the live image.

In an example, determining the spatial correspondence of the first image sensor may comprise analysing an anatomy of the target in the live image and comparing to an anatomy in the first radiographic image.

In an example, the first image sensor may comprise a set of radio frequency beacons, and the apparatus may comprise a radio frequency receiver, and wherein determining the spatial correspondence of the first image sensor may comprise analysing signals emitted by the radio frequency beacons which are received by the radio frequency receiver.

In an example, the controller may be configured to determine a pose of the target captured in the first radiographic image, and the user interface comprises displaying information on the pose in the first radiographic image.

In an example, the controller may be configured to determine a pose correction based on the determined pose in the first radiographic image, and the user interface comprises displaying information corresponding to the determined pose correction.

In an example, the first image sensor and the controller may be configured to communicate wirelessly.

In an example, the radiation generator may be provided on a moveable head part of the apparatus.

In an example, the display may be provided on the moveable head.

In an example, the second image sensor may be provided on the moveable head.

In a related aspect of the invention there may be provided a method for controlling a radiographic imaging apparatus. The method comprises performing a first data acquisition comprising capturing a first positional image of a target at substantially the same time as capturing a first radiographic image of the target, and prior to performing a second data acquisition, displaying a user interface comprising displaying a live image of the target currently being captured, the first positional image and the first radiographic image.

It will be appreciated that the invention also extends to method aspects corresponding to the apparatus aspects outlined above.

These and other aspects of the present invention will be apparent from and elucidated with reference to the embodiment(s) described hereinafter.

### BRIEF DESCRIPTION OF DRAWINGS

The embodiments of the present inventions may be best understood with reference to the accompanying figures, in which:
Fig. 1 shows a schematic of a radiography apparatus;
Fig. 2 shows an example display and user interface;
Fig. 3 shows another example user interface;
Fig. 4 shows an example image transformation; and
Fig. 5 show another example user interface.

### DETAILED DESCRIPTION OF EMBODIMENTS

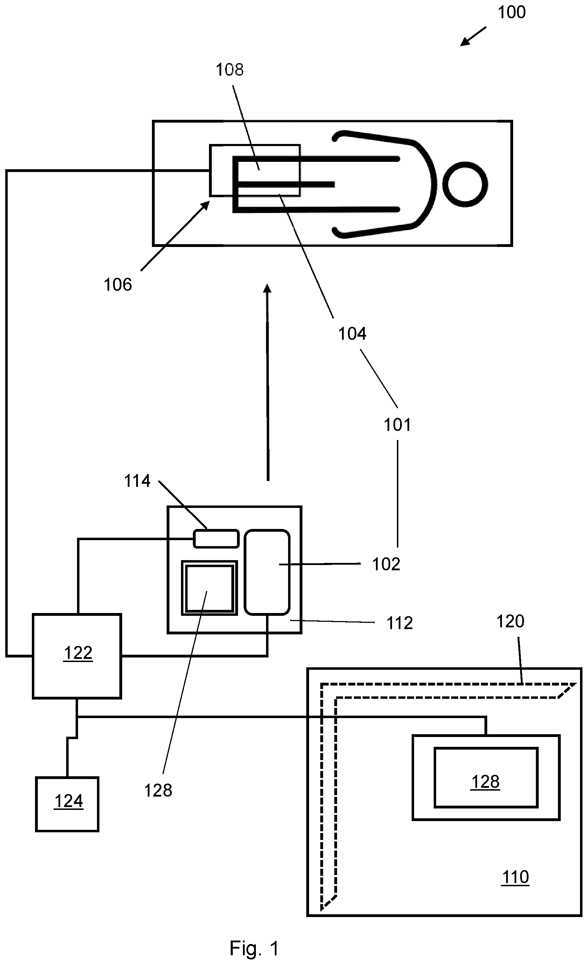

With reference to Fig. 1, there is shown an example radiography apparatus (or system) 100. The radiography apparatus 100 is based around the emission of ionising radiation and the subsequent imaging of that radiation. For example, the radiography apparatus 100 may be embodied as an Xray imaging system, a computed tomography (CT) scan system, a positron emission tomography (PET) scan, and other variants of imaging systems using ionizing radiation.

Suitably, the apparatus 100 comprises a radiographic imaging device 101; for example, comprising an ionising radiation generator 102 and a radiographic image sensor 104, also termed a first image sensor herein. For example, the radiation generator 102 may be configured to generate X-rays and the first image sensor 104 correspondingly configured to capture an X-ray image. If the generator 102 is configured to generate a different form of ionising radiation, the image sensor 104 is configured accordingly. As will be familiar to those in the art, the arrangement of the radiographic imager 102, 104 defines a target area 106 in which a target 108, such as a patient, or part thereof, may be positioned in order to capture a radiographic image based on the penetration of radiation through the target 108 in the target area 106. Put another way, it may be considered that the image sensor 104 is configured to capture a radiographic image of the target area 106.

In some examples, the radiographic imager 102, 104 are provided in a fixed arrangement, with the target 108 being moved with respect to the imaging components 102, 104 to allow for imaging of different parts of the target 108. Accordingly, the target area 106 to be imaged may be considered fixed.

In other examples, the radiographic imager 102, 104 may be coupled to suitable movement means such that one or both of the first imaging sensor 104 and radiation generator 102 may be moved relative to the target 108. That is, the target 108 may be considered substantially fixed in position, while the target area 106 to be imaged may be re-positioned according to the moveable arrangement between the first imaging sensor 104 and radiation generator 102. For example, the imaging sensor 104 may be coupled to one or more rails to provide lateral motion, while the generator 102 may be coupled to a moveable arm.

In another example, the radiation generator may be provided on a suitably moveable head part, or tube-head, 112. Meanwhile, the image sensor 104 may be provided as a separate unit configured to communicate wirelessly with other components of the apparatus 100. Suitably, the tube head 112 may be aligned with the target 108 and the image sensor 104 freely positioned behind the target 108 to capture an image of the ionising radiation.

In one example, the apparatus 100 may comprise a control station 110, from which control parameters and functions of the radiographic imaging device 101 may be set. The control station is disposed separated from the target area 106 of the radiographic imaging device 101 so as to limit the potential for exposure of an operator of the apparatus 100 to radiation. In some examples the control station 110 is shielded from the imaging device 101 (more specifically, the ionising radiation generator 102 and target area 106 therefor) by a radiation shield 120.

The apparatus may comprise a display 128 for viewing images captured by the radiographic imaging device 101. The display 128 may be provided at the control station 110, or in some examples may be provided on the tube head 112.

The apparatus 100 comprises a second image sensor 114 having a field of view which encompasses the target area 106. That is, the second image sensor 114 may be provided on the apparatus 100 to capture images of the target 108 within the target area 106, from which an operator may instruct adjustment of the target 108 and/or apparatus 100 in order to allow for imaging of the correct part of the target 108.

The second imaging sensor 114 may be configured to capture light at any suitably wavelength which allows for positioning the target 108. In one example the second image sensor may be configured to capture visible light; suitably, in such examples the second image sensor 114 may be embodied as an RGB camera. In another example the second image sensor may be configured to capture infra-red light, and may therefore be embodied as an infra-red camera. In yet another example, the second image sensor 114 may also be configured to capture depth information; suitably, in such examples the second image sensor 114 may be part of an RGB depth camera.

In some examples, the second image sensor 114 is integrated as part of the radiographic imager. That is, as a component of the respective part of the imaging components, whether that's the first imaging sensor 104, the x-ray generator 102, or another related component thereof. In the present example the second image sensor is integrated as part of the tube head 112 of the apparatus 100, such that its position substantially corresponds to the position of the radiation generator 102, and its field of view substantially corresponds with the direction of radiation emission from the generator 102.

The apparatus 100 comprises a controller 122 (e.g., a processor) configured to control an operating state of the imaging device. One example operating state is an image capturing state, or a data acquisition state, whereby the device is currently active in capturing an image; for example, controlling the radiation generator 102 to emit radiation and the image sensor 104 to capture an image of the received radiation after travelling through the target area 106.

In the present examples the controller 122 is configured to control a first data acquisition comprising controlling the radiographic imager to capture a first radiographic image of the target. That is, the controller 122 controls the radiation generator 102 to emit radiation and the first image sensor 114 to record an image based on the received radiation. At substantially the same time, the controller 122 controls the second image sensor 114 to also capture a first positional image of the target 108. That is, the first data acquisition also comprises capturing second image sensor 114 data.

Suitably, the first data acquisition may be stored in a memory 124. The memory 124 may be local to the apparatus 100 - e.g., an internal memory storage - or it may be external. In the case of external memory 124 the apparatus 100 may comprise suitable transceiver circuitry for wireless communication with the external memory 124.

It will be appreciated however that the first data acquisition may not result in a suitable radiographic image of the target 108. Many factors can influence the resultant image quality, however a primary concern to radiographers is target pose. It may thus be desirable to take a repeat image of the target 108 at the correct pose.

Suitably, prior to a second data acquisition, when a radiographer may be attempting to reposition the target, the controller controls the display 128 to display a user interface for aiding the radiographer. The user interface may be displayed in response to receiving a user input from the radiographer, or may be displayed automatically following the first data acquisition.

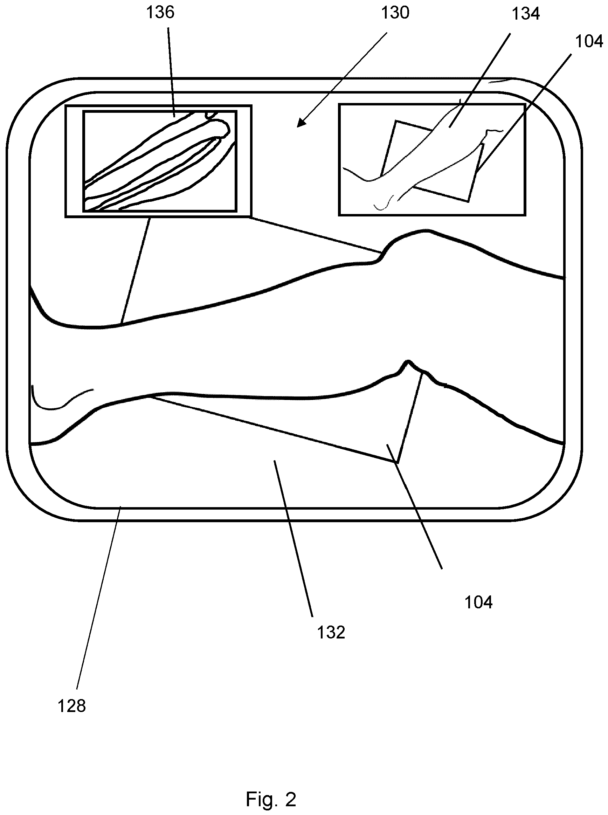

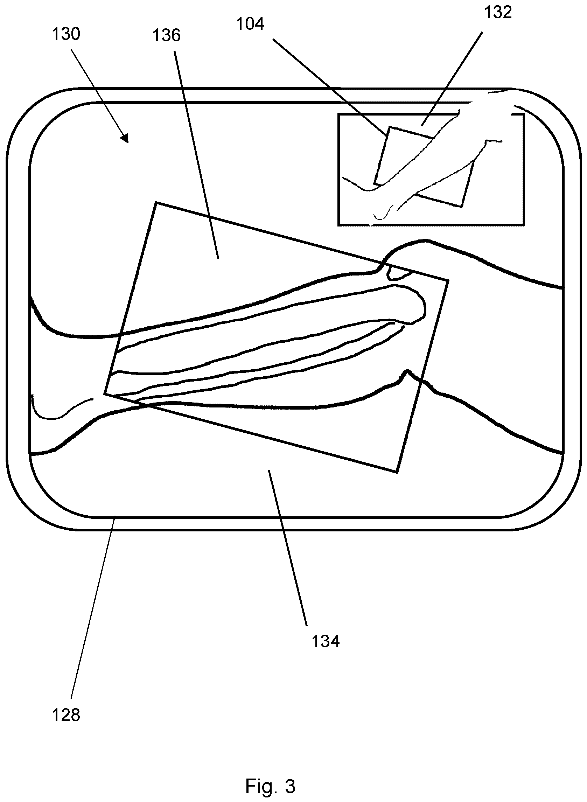

With reference to Figs. 2 and 3, there is shown examples of possible user interfaces. Here, the controller 122 controls the display 128 to display a user interface 130 comprising displaying a live image (or current scene) 132 of the target currently being captured by the second image sensor, the first positional image 134, and the first radiographic image 136. Put another way, there is provided a guided user interface (GUI) which allows to display an augmented camera image corresponding to the previous image acquisition instead or in addition to the current camera image during re-positioning for the retake.

Suitably, the current patient's pose during re-positioning for a retake is shown on the display 128. If the radiographer needs to compare the patient's pose corresponding to the previous image acquisition to the current pose, the GUI 130 allows to display the corresponding previous positioning image on the display while showing the current scene. The current scene 132 may be shown in a subwindow in a portion of the UI 130. In another example, the GUI 130 may comprise displaying the first positional image 134 overlaid, semi-transparently, onto the current scene. In yet another example, as shown by Fig. 3, the GUI 130 may comprise overlaying the first radiographic image 136 (e.g., X-ray) on the first positional image 134; either fully or semi-transparently. In another example the first radiographic image 136 may be overlaid on the current scene 132. In yet another example, the GUI 130 may comprise displaying a three dimensional rendering of the first positional image overlaid on one of the current scene 132 and the first radiographic image 136.

It will however be appreciated that in order to overlay the first radiographic image 136 that a spatial correlation must be established between the first image sensor 104 and second image sensor 114. In other words, the controller 122 may be configured to determine a spatial correlation of the first image sensor 104 with respect to at least one of the live scene being captured 132 and the previously captured first positional image 134. A number of approaches may be employed.

In one example, the first image sensor 104 may be localized due to known geometrical constellations, e.g. when placing it below the table in a tray of known location / orientation relative to the second image sensor 114. In this case, the first image sensor 104 does not need to be visible, but the second image sensor 114 needs to be either calibrated or located in a known geometrical constellation to the first image sensor 104.

In another example, suitable for when the first image sensor 114 will be visible to the second image sensor 114 - i.e., an image captured by the second image sensor 114 comprises an at least partial image of the first image sensor 104 - then the first image sensor 104 may comprise one or more markers 138. The markers may be particular shapes, colours, QR codes, and so on, and may be positioned on the first image sensor 104 in a known geometric arrangement (e.g., on the corners). Suitably, the controller 122 may perform image analysis to identify the position of the markers in the image. The analysis may be performed on both the live image 132 being captured by the second image sensor 114, or the first positional image 134 previously captured.

In another example, determining the spatial correspondence of the first image sensor 104 comprises analysing an anatomy of the target 108. That is, the controller 122 may perform image analysis on the first radiographic image 136 to determine an orientation of the anatomy with respect to the first image sensor 104, and also perform image analysis on the first positional image 134 to determine an orientation of the same anatomy with respect to the first image sensor 104. For example, if the first radiographic image 136 shows a tibia and femur either side of a knee joint, the controller 122 may determine an orientation of an upper / lower leg corresponding to the tibia and femur, and then determine an orientation and position of the upper and lower leg in the first positional image 134 that was taken at the same time as the first radiographic image 136. The controller 122 may then determine the spatial position of the first image sensor 104 within the first positional image 134, and then overlay the first radiographic image 136 and first positional image 134.

Analysing the anatomy of the target may utilise a number of approaches but it is particularly envisaged that the controller 122 may run a computer vision, or machine learning, algorithm to detect the anatomy and thereby localise the first image sensor 104 with respect to the first positional image 132. It will be appreciated that a machine learning approach to anatomy localization can be realized by training on a suitably designed neural networks to e.g. localize / segment the respective structures in the images.

In yet another example, the first image sensor 104 may comprise a set of radiofrequency beacons 140. The beacons 140 may emit at radiofrequency, for example a Bluetooth frequency or an ultra-wide band frequency. Different beacons 140 may be arranged to emit at slightly different frequencies, and/or in a well-defined temporal pattern. It will be appreciated that the beacons 140 are of course arranged in a predefined spatial pattern, such as the corners (like the markers 138).

The apparatus 100 may comprise one or more radiofrequency receivers coupled to the controller 122. Suitably, determining the spatial correspondence of the first image sensor 104 within the first positional image 134, may comprise analysing the signals received from the radio frequency beacons 140 to determine the spatial arrangement from the temporal pattern. For example, by triangulation. Advantageously in this arrangement this first image sensor 104 does not need to be visible.

In yet another example, a spatial relationship between the radiated area on the first image sensor 104 and the corresponding image captured by the second image sensor 114 may be established by retrieving a security identifier, collimation parameters, and a plane of the first image sensor 104 relative to radiation generator 102 (e.g. taking the tube-angulation into account) from the system, calculating corner points of the radiated region on the first image sensor 104 in the first positional image 134 (using known spatial correspondence to the radiation generator 102), manually / automatically localizing the collimated area in the first radiographic image and deriving the corresponding corner-points from this information, and resolving the potential ambiguity regarding discrete rotations when finding the correspondence between the corner points in the radiographic image and the positional image by automatically detecting a reference direction in both image (e.g. direction towards the head) using computer vision or machine learning.

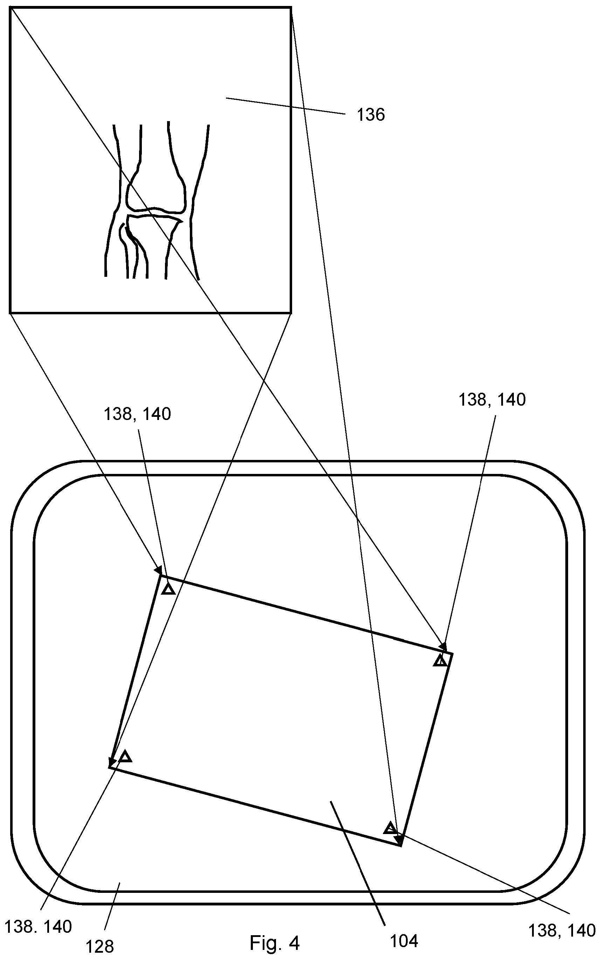

Having localized the first image sensor 104 relative to the first positional image 134 (or the live captured image 132), a geometrical relationship between the first radiographic image 136 and an image captured by the second image sensor 114 can be established by determining the affine transformation which maps the corners of the detector pixel-matrix to the corresponding points in the camera image. This is demonstrated in Fig. 4. The radiographic image 136 pixel-matrix can be transformed onto the image from the second image sensor 114 using this affine transformation. The transformed radiographic image 136 may be restricted to the collimated area using the known / in the X-ray pixel-matrix detected shutter configuration. Finally, the second image sensor image 132/134 and the radiographic image 136 can be fused by e.g. transparent overlays.

Furthermore, the visualization may be refined by using a perspective transformation instead of an affine one if, e.g. using also depth information (via an RGBD camera) or system parameters (e.g. SID, tube angulation).

In an example where a depth of the target 108 anatomy may be determined (using e.g., a depth camera), the depth surfaces of the anatomy corresponding to the current patient pose and the pose of the previous acquisition can be registered. The deviation between the poses (e.g. 8° difference of leg-rotation, 10° difference in flexion) can be derived from the registration transformation and anatomical knowledge (e.g. by detecting landmarks on the anatomy by a correspondingly trained neural network).

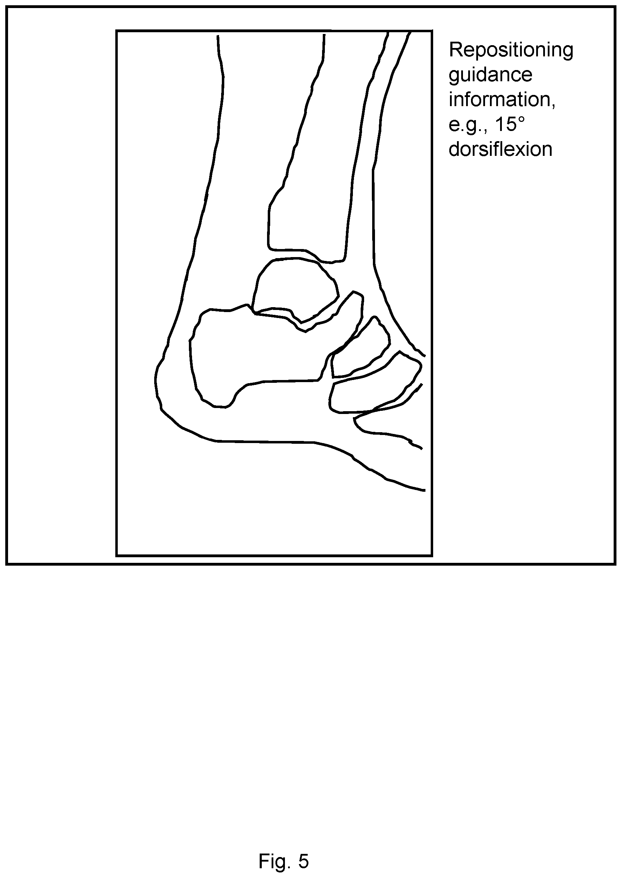

In an example, such measurements of a pose of the target 108, based on the target as captured in the first radiographic image 136, may be displayed on the user interface as shown in the example of Fig. 5. Put another way, when displayed in the GUI 130, the first radiographic image 136 may comprise information on the pose of the target 108.

Suitably, the radiographer may be presented with quantitative information on pose as part of the GUI to supplement any qualitative visualisation. That is, the radiographer may be presented with results from automatic assessment of positioning quality of the previous radiographic image from which to directly infer which positioning degrees of freedom need to be adapted, in which direction and how much, etc. Put another way, the controller 122 may determine a pose correction based on the determined pose in the first radiographic image, and control to display information corresponding to the determined pose correction (as part of the first radiographic image 136).

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. A radiography apparatus (100), comprising:
a radiographic imager (101) configured to capture a radiographic image of a target (108), the radiographic imager comprising a radiation generator (102) and a first image sensor (104);
a positional imager comprising a second image sensor (114) configured to capture an image of the target;
a controller (122) configured to control a first data acquisition comprising controlling the second image sensor to capture a first positional image (134) of the target at substantially the same time as controlling the radiographic imager to capture a first radiographic image of the target (136), and to store the captured first images in a memory (124); and
a display (128);
wherein, prior to a second data acquisition, the controller controls the display to display a user interface (130) comprising displaying a live image (132) of the target currently captured by the second image sensor, the first positional image and the first radiographic image.

2. The apparatus of claim 1, wherein displaying the first radiographic image comprises overlaying the first radiographic image and a corresponding area of the first positional image.

3. The apparatus of any of claims 1 or 2, wherein the positional imager comprises a depth sensor.

4. The apparatus of claims 2 and 3, wherein the user interface comprises displaying a three dimensional rendering of the first positional image on top of the first radiographic image.

5. The apparatus of any preceding claim, wherein the user interface comprises displaying the currently captured live image overlaid with first positional image.

6. The apparatus of any preceding claim, wherein the controller is configured to determine a spatial correspondence of the first image sensor with respect to the live image being captured.

7. The apparatus of claim 6, wherein the first image sensor comprises one or more markers (138), and wherein determining the spatial correspondence of the first image sensor comprises determining the location of the markers within the live image.

8. The apparatus of claim 6, wherein determining the spatial correspondence of the first image sensor comprises analysing an anatomy of the target in the live image and comparing to an anatomy in the first radiographic image.

9. The apparatus of claim 6, wherein the first image sensor comprises a set of radio frequency beacons (140), and the apparatus comprises a radio frequency receiver, and wherein determining the spatial correspondence of the first image sensor comprises analysing signals emitted by the radio frequency beacons which are received by the radio frequency receiver.

10. The apparatus of any preceding claim, wherein the controller is configured to determine a pose of the target captured in the first radiographic image, and the user interface comprises displaying information on the pose in the first radiographic image.

11. The apparatus of claim 10, wherein the controller is configured to determine a pose correction based on the determined pose in the first radiographic image, and the user interface comprises displaying information corresponding to the determined pose correction.

12. The apparatus of any preceding claim, wherein the first image sensor and the controller are configured to communicate wirelessly.

13. The apparatus of any preceding claim, wherein the radiation generator is provided on a moveable head part of the apparatus, and wherein the second image sensor is also provided on the moveable head.

14. The apparatus of claim 13, wherein the display is provided on the moveable head.

15. A method for controlling a radiographic imaging apparatus, comprising:
performing a first data acquisition comprising capturing a first positional image of a target at substantially the same time as capturing a first radiographic image of the target; and
prior to performing a second data acquisition, displaying a user interface comprising displaying a live image of the target currently being captured, the first positional image and the first radiographic image.
